# EUROPEAN PATENT APPLICATION

(11) **EP 3 975 198 A1**
(43) Date of publication of application: **30.03.2022**
(21) Application number: 20831564.8
(22) Date of filing: 08.06.2020
(51) Int. Cl.: G16H 30/40

(54) **DIAGNOSIS SUPPORT PROGRAM, DIAGNOSIS SUPPORT SYSTEM, AND DIAGNOSIS SUPPORT METHOD**

(30) Priority: 24.06.2019 JP 2019116793
(71) Applicant: Sony Group Corporation, Tokyo 108-0075 (JP)
(72) Inventor: SUGIE, Yuki, Tokyo 108-0075 (JP)
(74) Representative: D Young & Co LLP
(86) International application number: PCT/JP2020/022475
(87) International publication number: WO 2020/261955

(57) **Abstract**

A diagnosis support program is a program for causing a computer to perform: an image processing procedure of performing predetermined image processing on a pathological image captured by an image-capturing device; and an output processing procedure of outputting identification information for identifying a processed portion and an unprocessed portion of the image processing on the pathological image.

## Description

### Field

The present disclosure relates to a diagnosis support program, a diagnosis support system, and a diagnosis support method.

### Background

Besides clinical diagnosis, in which clinicians diagnose patients, major conventional diagnosis methods in medical facilities include, for example, pathological diagnosis, in which pathologists make diagnoses on pathological images that are captured images of an observation object (sample) collected from a patient. Pathological diagnosis is very important because its diagnosis results significantly affect the treatment plan for the patient and the like.

When viewing pathological images in pathological diagnosis, the pathologist may perform predetermined image processing (such as color correction, edge enhancement, and contrast correction, for example) on the pathological images according to the performance of the monitor, the pathologist's preference, or the like.

### Summary

### Technical Problem

However, in general, pathological images have high resolution, and therefore the predetermined image processing on the pathological images is often not completed instantly. Thus, the pathologist may view pathological images in which a processed portion and unprocessed portion of the predetermined image processing are mixed. There has been a concern that a wrong diagnosis may occur because the pathologist cannot reliably distinguish the processed portion and unprocessed portion in the pathological images.

The present disclosure has been made in view of the above circumstances and proposes a diagnosis support program, a diagnosis support system, and a diagnosis support method that can improve the accuracy of pathological diagnosis using a pathological image.

### Solution to Problem

To solve the problems described above, a diagnosis support program causes a computer to perform: an image processing procedure of performing predetermined image processing on a pathological image captured by an image-capturing device; and an output processing procedure of outputting identification information for identifying a processed portion and an unprocessed portion of the image processing on the pathological image.

### Brief Description of Drawings

FIG. 1 is an entire configuration diagram of a diagnosis support system according to a first embodiment.
FIG. 2 is a flow chart illustrating processing by a viewer according to the first embodiment.
FIG. 3 is a diagram schematically illustrating a first example of pathological images according to the first embodiment.
FIG. 4 is a diagram schematically illustrating a second example of pathological images according to the first embodiment.
FIG. 5 is a diagram schematically illustrating a third example of pathological images according to the first embodiment.
FIG. 6 is a diagram schematically illustrating a fourth example of pathological images according to the first embodiment.
FIG. 7 is a diagram schematically illustrating a fifth example of pathological images according to the first embodiment.
FIG. 8 is a diagram schematically illustrating a sixth example of pathological images according to the first embodiment.
FIG. 9 is a diagram schematically illustrating an example of a UI for performing predetermined image processing on a pathological image according to the first embodiment.
FIG. 10 is a diagram schematically illustrating an example of display for identifying an unprocessed portion in a pathological image according to the first embodiment.
FIG. 11 is an entire configuration diagram of a diagnosis support system according to a second embodiment.
FIG. 12 is a diagram for illustrating an image-capturing process according to the second embodiment.
FIG. 13 is a diagram for illustrating a generation process of partial images (tile images) in the second embodiment.
FIG. 14 is a diagram for illustrating a pathological image according to the second embodiment.
FIG. 15 is a diagram for illustrating a pathological image according to the second embodiment.
FIG. 16 is a diagram schematically illustrating an example of displaying a pathological image according to the second embodiment.
FIG. 17 is an entire configuration diagram of a diagnosis support system according to a third embodiment.
FIG. 18 is a hardware configuration diagram illustrating an example of a computer for realizing functions of the viewer.

### Description of Embodiments

Embodiments of the present disclosure will be described in detail below with reference to the drawings. Note that, in the following embodiments, overlapping descriptions will be omitted as appropriate by giving the same reference characters to the same components.

The present disclosure will be described in the following order of items.

### <First Embodiment>

1. Configuration of System according to First Embodiment
2. Processing Procedure by Viewer according to First Embodiment
3. First to Sixth Examples of Pathological Images according to First Embodiment and the like

### <Second Embodiment>

4. Configuration of System according to Second Embodiment
5. Description of Tile Images in Second Embodiment
6. Example of Displaying Pathological Image according to Second Embodiment

### <Third Embodiment>

7. Configuration of System according to Third Embodiment

### <Other Embodiments>

### <First Embodiment>

### [1. Configuration of System according to First Embodiment]

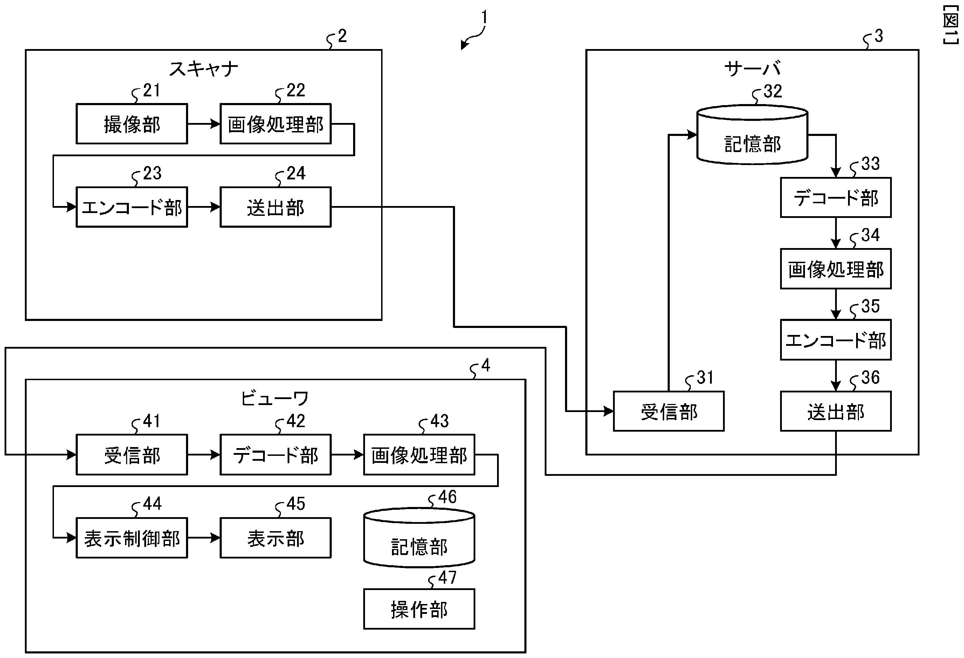

First, a diagnosis support system 1 according to a first embodiment will be described with reference to FIG. 1. FIG. 1 is an entire configuration diagram of the diagnosis support system 1 according to the first embodiment. As illustrated in FIG. 1, the diagnosis support system 1 includes a scanner 2, a server 3 (information processing device), and a viewer 4 (information processing device). Note that the scanner 2, the server 3, and the viewer 4 each include a communication unit (not illustrated) realized by a NIC (Network Interface Card) or the like, are connected to a communication network (not illustrated) in a wired or wireless manner, and can send and receive information to/from each other via the communication network. Note that arrows in the figure indicate main flows of information and sending and receiving of information can also be performed at portions without arrows.

In conventional techniques, in such a pathological diagnosis (digital pathology imaging (DPI)) system, when predetermined image processing on a pathological image is not completed instantly, for example, quick preview display of unprocessed portions of the pathological image by quick development processing has been used in order to reduce the waiting time experienced by an observer (such as a pathologist, the same applies hereinafter). However, there has been a concern that a wrong diagnosis may occur because pathologists, who may make a diagnosis on one pathological image in several seconds, cannot reliably distinguish a processed portion and an unprocessed portion in the pathological image, in which such quick preview display is mixed. Thus, the following will describe a technique of providing a pathological image in which a pathologist can reliably distinguish a processed portion and an unprocessed portion.

The scanner 2 is, for example, an image-capturing device that has the function of an optical microscope, captures an image of an observation object (sample) contained in a glass slide, and acquires a pathological image, which is a digital image. Note that the observation object is, for example, tissues or cells collected from a patient, such as a piece of flesh of an organ, saliva, or blood. The scanner 2 includes an image capturing unit 21, an image processing unit 22, an encoding unit 23, and a sending unit 24.

The image capturing unit 21 captures an image of the observation object contained in a glass slide and outputs an image-capturing signal. The image processing unit 22 performs basic image processing (such as demosaicing, for example) on the image-capturing signal output by the image capturing unit 21.

The encoding unit 23 encodes the pathological image on which the image processing is performed by the image processing unit 22. The sending unit 24 sends the pathological image encoded by the encoding unit 23 to the server 3.

The server 3 is a computer device that performs storage, processing and the like of the pathological image captured by the scanner 2. Also, when accepting a request for viewing the pathological image from the viewer 4, the server 3 retrieves the pathological image and sends the retrieved pathological image to the viewer 4. The server 3 includes a receiving unit 31, a storage unit 32, a decoding unit 33, an image processing unit 34, an encoding unit 35, and a sending unit 36.

The server 3 executes a predetermined program to realize each function. Note that the program may be stored in the server 3 or may be stored in a storage medium such as a digital versatile disc (DVD), a cloud computer, or the like. Also, the program may be executed by a central processing unit (CPU) or a micro-processor (MPU) by using a random access memory (RAM) or the like as a workspace in the server 3, or may be executed by an integrated circuit such as an application-specific integrated circuit (ASIC) or a field programmable gate array (FPGA).

The receiving unit 31 receives the pathological image sent from the scanner 2 and stores it in the storage unit 32. The storage unit 32 is realized by, for example, a storage device such as a semiconductor memory device such as a RAM or a flash memory, a hard disk, or an optical disc. The storage unit 32 stores various programs, data, the pathological image received from the scanner 2, and the like.

The decoding unit 33 reads the pathological image from the storage unit 32 and decodes it. The image processing unit 34 performs predetermined image processing (such as color correction, edge enhancement, or contrast correction, for example, this may also be referred to simply as "image processing" hereinafter) on the pathological image decoded by the decoding unit 33 according to the performance of a display unit 45 of the viewer 4, the pathologist's preference, or the like.

The encoding unit 35 encodes the pathological image on which the image processing is performed by the image processing unit 34. The sending unit 36 sends the pathological image encoded by the encoding unit 35 to the viewer 4.

The viewer 4 is a computer device that is mainly used by the pathologist and displays a pathological image accepted from the server 3 to which a viewing request is sent, and is installed in a research institute or a hospital, for example. The viewer 4 includes a receiving unit 41, a decoding unit 42, an image processing unit 43, a display control unit 44, a display unit 45, a storage unit 46, and an operation unit 47.

The viewer 4 executes a predetermined program to realize each function. Note that the program may be stored in the server 3 or the viewer 4, or may be stored in a storage medium such as a DVD, a cloud computer, or the like. Also, the program may be executed by a CPU or an MPU by using a RAM or the like as a workspace, or may be executed by an integrated circuit such as an ASIC or an FPGA.

The receiving unit 41 receives the pathological image sent from the server 3. The decoding unit 42 decodes the pathological image received by the receiving unit 41.

The image processing unit 43 performs an image processing procedure of performing predetermined image processing (such as color correction, edge enhancement, or contrast correction, for example) on the pathological image decoded by the decoding unit 42 according to the performance of the display unit 45, the pathologist's preference, or the like. Note that the processing by the image processing unit 43 is performed (continued) even while the pathological image is displayed on the display unit 45.

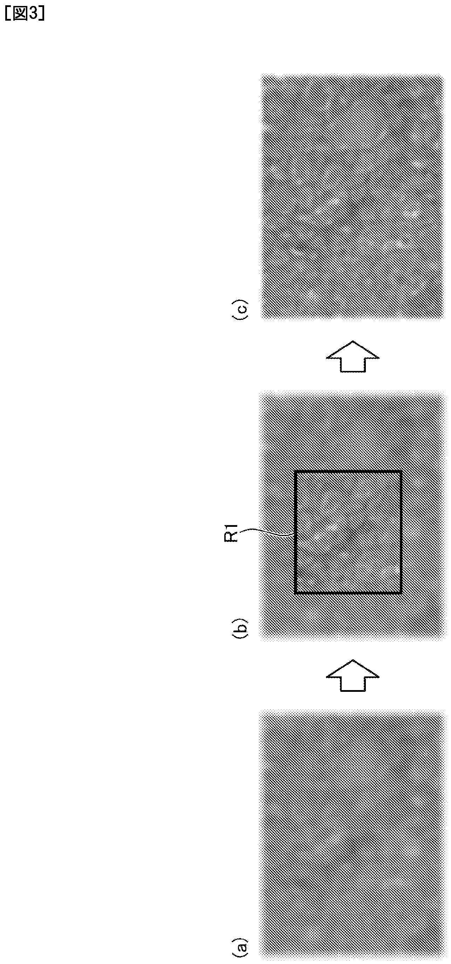

For example, the image processing procedure performs the image processing from a central portion of a region displayed on the display unit 45 in the pathological image (FIG. 3: details will be described later).

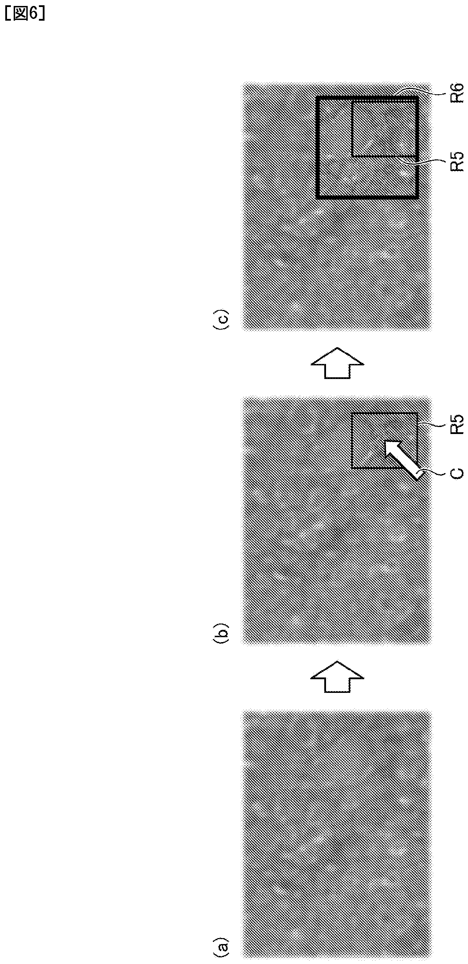

For example, the image processing procedure may also perform the image processing from a portion corresponding to a cursor position displayed on the display unit 45 in the pathological image (FIG. 6: details will be described later).

The display control unit 44 performs an output processing procedure of outputting identification information for identifying a processed portion and an unprocessed portion of the image processing on the pathological image. Note that the processing by the display control unit 44 is performed (continued) even while the pathological image is displayed on the display unit 45.

For example, the output processing procedure displays a boundary line as the identification information at the boundary between the processed portion and the unprocessed portion displayed on the display unit 45 (FIG. 3: details will be described later).

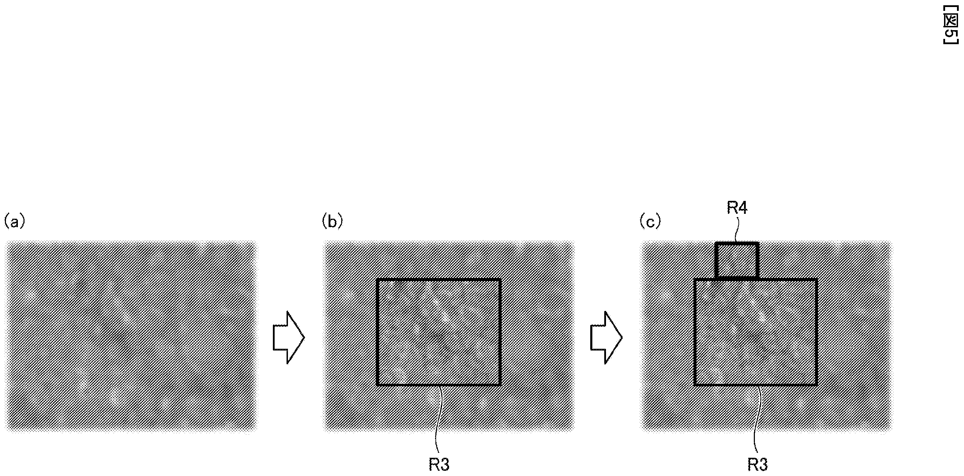

The output processing procedure may also highlight a new processed portion of the processed portion displayed on the display unit 45 by means of at least one of the color, thickness, or flashing of the boundary line surrounding it (FIG. 5: details will be described later).

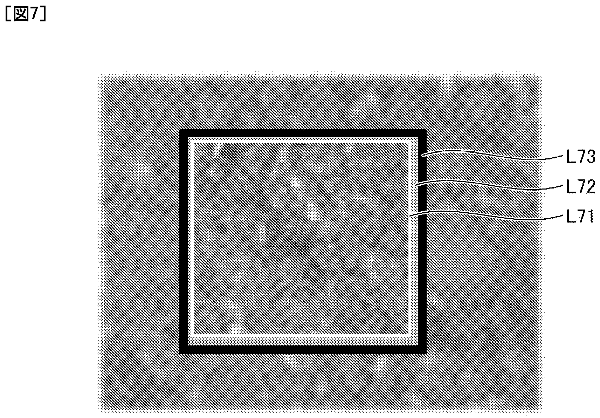

The output processing procedure may also display the boundary line by a line of a first line type positioned closer to the processed portion and a line of a second line type positioned closer to the unprocessed portion (FIG. 7: details will be described later).

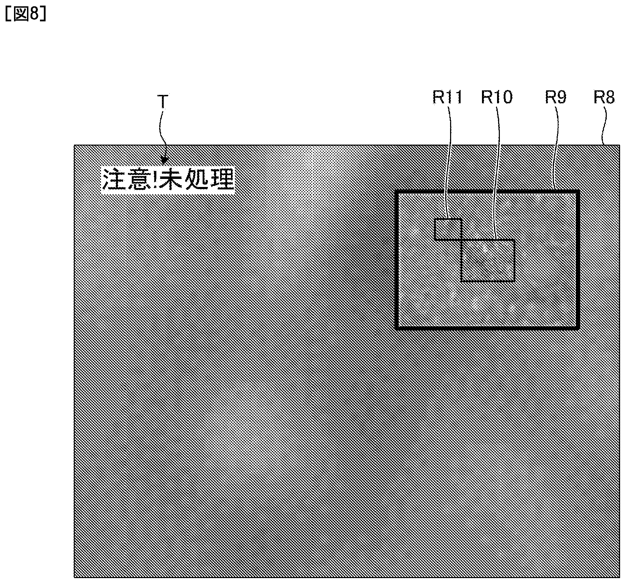

The output processing procedure may also display the entire pathological image in a portion of the display unit 45 and display a boundary line at the boundary between the processed portion and the unprocessed portion in the entire displayed pathological image in enlarging a portion of the pathological image on the display unit (FIG. 8: details will be described later).

The output processing procedure may also highlight a portion of the pathological image enlarged on the display unit 45 when the portion of the pathological image is the unprocessed portion (FIG. 8: details will be described later).

The output processing procedure may also indicate at least one of the processed portion and the unprocessed portion by means of a text indication on the display unit 45 (FIG. 8: details will be described later).

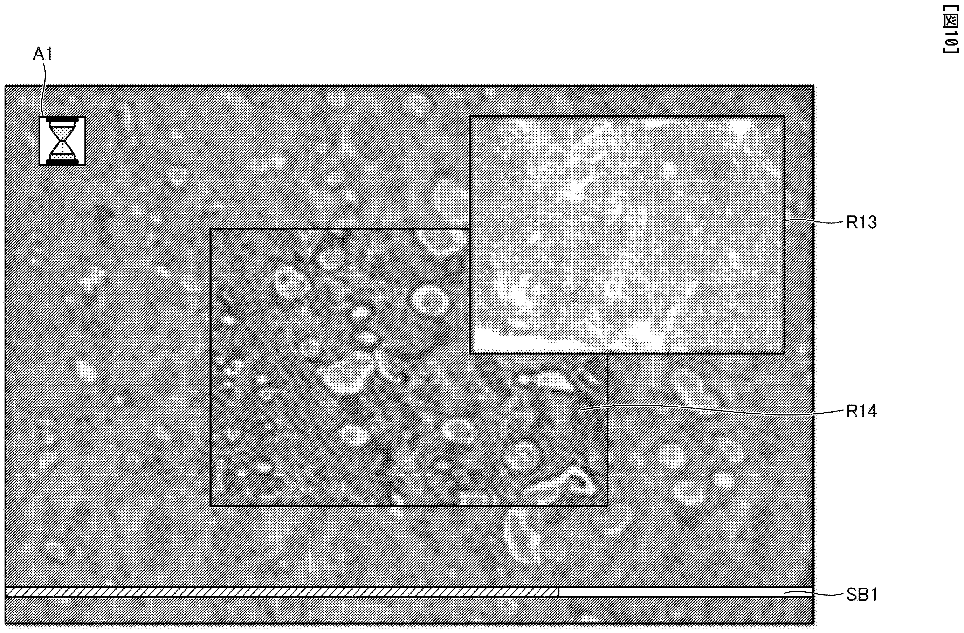

The output processing procedure may also indicate that the unprocessed portion is displayed by means of a diagrammatic indication on the display unit 45 (FIG. 10: details will be described later).

The output processing procedure may also perform display on the display unit 45 after performing predetermined image-quality degradation processing on the unprocessed portion. For example, by performing luminance reduction or gray-scaling as the predetermined image-quality degradation processing on the unprocessed portion, the observer can reliably notice the unprocessed portion.

The display unit 45 is a means for displaying information and has, for example, a screen for which liquid crystal, electro-luminescence (EL), a cathode ray tube (CRT), or the like is used. Also, the display unit 45 may be compatible with 4K or 8K and may be formed by a plurality of display devices. The display unit 45 displays information (such as an image) according to control by the display control unit 44.

The storage unit 46 is realized by, for example, a storage device such as a semiconductor memory device such as a RAM or a flash memory, a hard disk, or an optical disc. The storage unit 46 stores various programs, data, the pathological image received from the server 3, and the like.

The operation unit 47 is a means for being operated by a user of the viewer 4 (such as a pathologist, the same applies hereinafter) and is, for example, a mouse, a keyboard, a touch panel, or the like.

Note that, although both of the image processing unit 34 of the server 3 and the image processing unit 43 of the viewer 4 can perform the predetermined image processing in the present embodiment, there is no limitation in this regard. For example, if the viewer 4 has a sufficient computing ability, only the viewer 4 may perform the predetermined image processing. Alternatively, for example, if the viewer 4 does not have a sufficient computing ability, only the server 3 may perform the predetermined image processing. Also, although the present disclosure will be described by assuming that the viewer 4, out of the server 3 and the viewer 4, has the function of outputting the identification information for identifying the processed portion and the unprocessed portion of the predetermined image processing in the pathological image, there is no limitation in this regard, and the server 3 may have a such function.

### [2. Processing Procedure by Viewer according to First Embodiment]

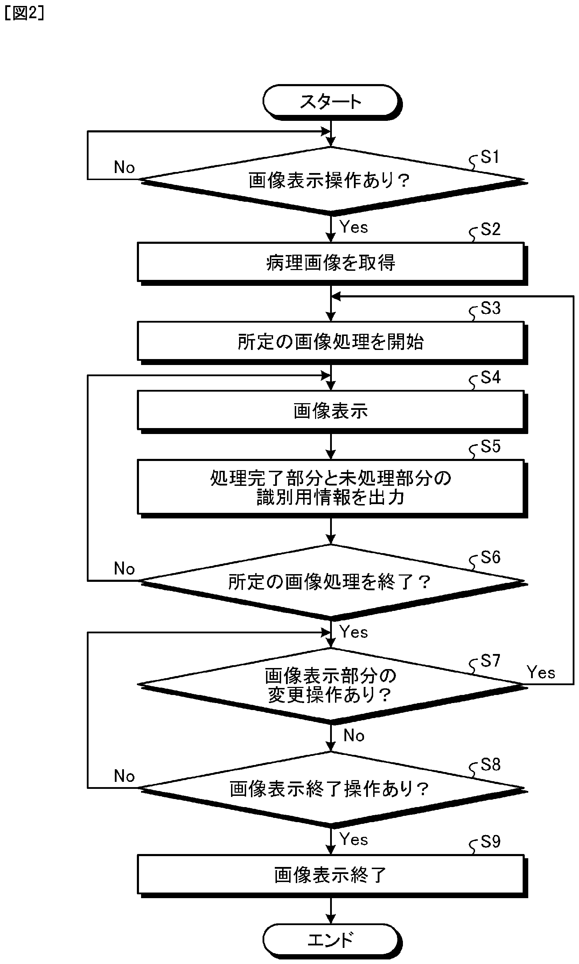

Next, processing by the viewer 4 according to the first embodiment will be described with reference to FIG. 2. FIG. 2 is a flow chart illustrating processing by the viewer 4 according to the first embodiment. Note that, in the following, description of some pieces of processing such as processing by the decoding unit 42 may be omitted for simplicity of description.

First, in step S1, the viewer 4 determines whether an image displaying operation, that is, an operation by a pathologist or the like via the operation unit 47 for displaying a pathological image has occurred, and if Yes, the process proceeds to step S2, and if No, the process returns to step S1.

In step S2, the receiving unit 41 acquires the pathological image from the server 3.

Next, in step S3, the image processing unit 43 starts predetermined image processing (such as color correction, edge enhancement, or contrast correction, for example) on the pathological image.

Next, in step S4, the display control unit 44 displays the pathological image on the display unit 45.

Next, in step S5, the display control unit 44 performs an output processing procedure of outputting identification information for identifying a processed portion and an unprocessed portion of the image processing on the pathological image.

Next, in step S6, the image processing unit 43 determines whether the predetermined image processing has ended, and if Yes, the process proceeds to step S7, and if No, the process returns to step S4. That is, during the loop processing of step S4 → step S5 → No in step S6 → step S4..., the processed portion and the unprocessed portion are displayed on the display unit 45 as the pathological image in a mixed manner, as well as the boundary line or the like for identifying the processed portion and the unprocessed portion. In this manner, the observer can reliably distinguish the processed portion and the unprocessed portion of the image processing on the pathological image (details will be described later).

In step S7, the viewer 4 determines whether an operation of changing the image display portion has occurred, that is, whether an operation by the observer via the operation unit 47 for changing the region of the pathological image displayed on the display unit 45 has occurred, and if Yes, the process returns to step S3, and if No, the process proceeds to step S8.

In step S8, the viewer 4 determines whether an image display ending operation, that is, an operation by the pathologist or the like via the operation unit 47 for ending the display of the pathological image has occurred, and if Yes, the process proceeds to step S9, and if No, the process returns to step S7.

In step S9, the viewer 4 ends the image display, that is, the display of the pathological image on the display unit 45.

### [3. First to Sixth Examples of Pathological Images according to First Embodiment and the like]

FIG. 3 is a diagram schematically illustrating a first example of pathological images according to the first embodiment. FIGS. 3(a) to (c) illustrate pathological images displayed on the display unit 45. FIG. 3(a) is a pathological image before the predetermined image processing is performed. FIG. 3(c) is a pathological image after the predetermined image processing is completed. The image processing procedure performed on the pathological image in FIG. 3(a) by the image processing unit 43 performs the predetermined image processing from a central portion of a region displayed on the display unit 45 in the pathological image. Since it can be generally considered that the observer often views the pathological image from its central portion, if the predetermined image processing is not completed instantly, performing the image processing from the central portion of the display region of the pathological image in this manner is convenient for the observer.

The output processing procedure performed by the display control unit 44 also displays a boundary line as the identification information at the boundary between the processed portion and the unprocessed portion displayed on the display unit 45. FIG. 3(b) is a pathological image in the middle of the predetermined image processing being performed. The inside of region R1 is a processed portion, and the outside of region R1 is an unprocessed portion.

For example, if the predetermined image processing is color correction processing, it is not easy to distinguish the processed portion and the unprocessed portion in the pathological image without the display of such a boundary line. Specifically, for example, in the case of a pathological image of an observation object stained with hematoxylin and eosin (HE), a lesion is determined by tones of color of purple to red-purple. In that case, when viewing the pathological image without the display of the boundary line, the pathologist may mistake the unprocessed portion of color correction for the processed portion and make a wrong diagnosis. The same applies to edge enhancement, and a normal cell may be determined as a cell with a lesion in a high-magnification image without performing appropriate edge enhancement processing, and thus the mixing of a processed portion and an unprocessed portion in a pathological image without the display of the boundary line can cause a wrong diagnosis.

In contrast, according to the present disclosure, by displaying the boundary line at the boundary between the processed portion and the unprocessed portion in the pathological image, the observer can reliably distinguish the processed portion and the unprocessed portion in the pathological image, and a wrong diagnosis can be avoided.

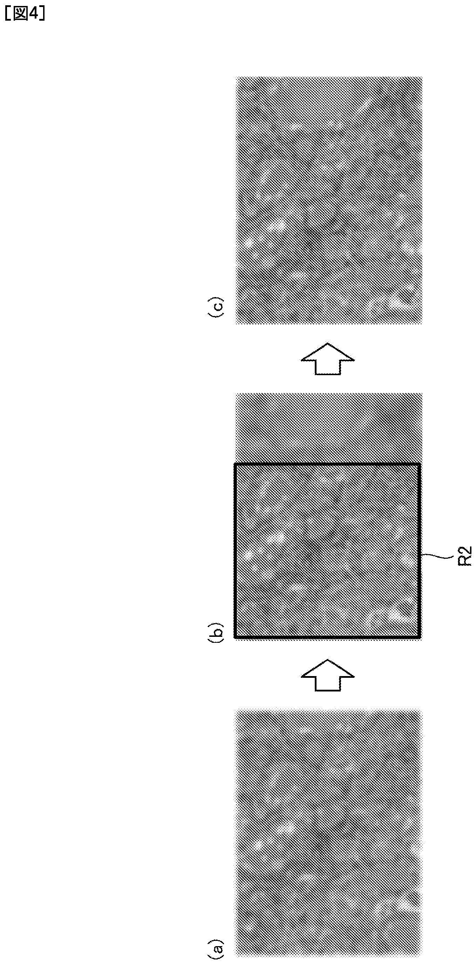

FIG. 4 is a diagram schematically illustrating a second example of pathological images according to the first embodiment. FIGS. 4(a) to (c) illustrate pathological images displayed on the display unit 45. FIG. 4(a) is a pathological image after the predetermined image processing is performed. Here, for example, it is assumed that the observer has moved the pathological image displayed on the display unit 45 to the left by operating the operation unit 47 (Yes in step S7 in FIG. 2).

Thus, as illustrated in FIG. 4(b), region R2 on the left side on the display unit 45 is a processed portion, and the outside of region R2 on the right side is an unprocessed portion. The output processing procedure performed by the display control unit 44 displays a boundary line between the inside and outside of region R2. In this manner, according to the present disclosure, by displaying the boundary line at the boundary between the processed portion and the unprocessed portion in the pathological image, the observer can reliably distinguish the processed portion and the unprocessed portion in the pathological image, and a wrong diagnosis can be avoided. Thereafter, when the predetermined image processing on the unprocessed portion outside region R2 in FIG. 4(b) is completed, the pathological image after the predetermined image processing is completed is on the entire screen as illustrated in FIG. 4(c).

FIG. 5 is a diagram schematically illustrating a third example of pathological images according to the first embodiment. FIGS. 5(a) to (c) illustrate pathological images displayed on the display unit 45. FIG. 5(a) is a pathological image before the predetermined image processing is performed. The image processing procedure performed on the pathological image in FIG. 5(a) by the image processing unit 43 performs the image processing from a central portion of a region displayed on the display unit 45 in the pathological image and displays a boundary line at the boundary between the processed portion and the unprocessed portion. FIG. 5(b) is a pathological image in the middle of the predetermined image processing being performed. The inside of region R3 is a processed portion, and the outside of region R1 is an unprocessed portion.

Here, the image processing procedure further performs the predetermined image processing on region R4 illustrated in FIG. 5(c), and in that process, the output processing procedure performs highlighting by displaying a thick boundary line surrounding region R4, which is a new processed portion. In this manner, by highlighting the new processed portion, the observer reliably recognizes the new processed portion (region R4) in the pathological image, and a wrong diagnosis can be avoided. Note that the manner of this highlighting is not limited to thickening the boundary line relative to another boundary line and may also be making the color of the boundary line different from the color of the other boundary line or flashing the boundary line.

FIG. 6 is a diagram schematically illustrating a fourth example of pathological images according to the first embodiment. FIGS. 6(a) to (c) illustrate pathological images displayed on the display unit 45. FIG. 6(a) is a pathological image before the predetermined image processing is performed. The image processing procedure for the pathological image in FIG. 6(a) performs the image processing from a portion corresponding to the position of a cursor (reference character C in FIG. 6(b)) displayed on the display unit 45 in the pathological image (hereinafter also referred to as a "cursor portion"). Since it can be considered that the observer is likely to look at the cursor portion of the pathological image, performing the image processing from the cursor portion is convenient for the observer.

The output processing procedure also displays a boundary line at the boundary between the processed portion and the unprocessed portion. In FIG. 6(b), the inside of region R5 is a processed portion, and the outside of region R5 is an unprocessed portion. Note that, in FIG. 6(c), a boundary line of region R6 including a new processed portion is highlighted in a manner similar to the boundary line of region R4 in FIG. 5(c).

FIG. 7 is a diagram schematically illustrating a fifth example of pathological images according to the first embodiment. As illustrated in FIG. 7, the output processing procedure displays a boundary line by line L71 of a first line type positioned closer to the processed portion (on the central side), line L73 of a second line type positioned closer to the unprocessed portion (on the outside), and line L72 between them. Here, lines L71, L72, and L73 have different degrees of darkness of color that increase in this order. The observer can reliably distinguish the processed portion and the unprocessed portion in the pathological image by recognizing in advance that the processed portion is on the lighter color side and the unprocessed portion is on the darker color side, and a wrong diagnosis can be avoided. Note that the way of varying the line types is not limited to varying colors and may also be varying between a solid line and a broken line or the like. Also, the number of lines forming the boundary line is not limited to three and may be two or four or more.

FIG. 8 is a diagram schematically illustrating a sixth example of pathological images according to the first embodiment. When the pathological image is enlarged and either the processed portion or the unprocessed portion is on the entire screen displayed on the display unit 45, the observer may be unable to easily determine which of the processed portion and the unprocessed portion is on the entire screen. Thus, as illustrated in FIG. 8, the output processing procedure enlarge a portion of the pathological image on the display unit 45 (region R8) by displaying the entire pathological image in a portion of the display unit 45 (region R9) and displays a boundary line at the boundary between the processed portion (inside region R10 in region R9) and the unprocessed portion (outside region R10 in region R9) in the entire displayed pathological image. Also, region R11 in region R9 corresponds to the enlarged portion (region R8).

In this manner, the observer can easily recognize that the enlarged portion (region R8) corresponds to region R11 in the entire portion (region R9) and is the unprocessed portion.

The output processing procedure also highlights (for example, displays with a bold frame) the enlarged portion (region R8), which is a portion of the pathological image. In this manner, the observer can further easily recognize that the enlarged portion (region R8) is the unprocessed portion.

The output processing procedure also indicates the unprocessed portion by means of a text indication (indication of "Attention! Unprocessed" with reference character T). In this manner, the observer can further easily recognize that the enlarged portion (region R8) is the unprocessed portion. Note that the text indication is not limited to being provided to the unprocessed portion and may be provided to the processed portion. Also, although the text indication is preferably provided by means of on-screen display (OSD), there is no limitation in this regard, and it may be provided by other means.

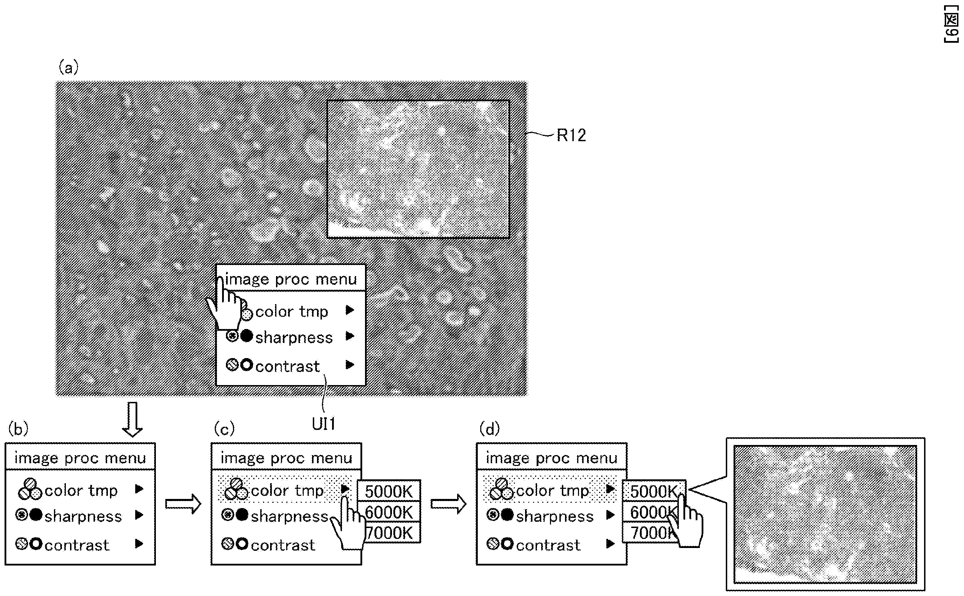

FIG. 9 is a diagram schematically illustrating an example of a user interface (UI) for performing predetermined image processing on a pathological image according to the first embodiment. On this screen, any of color temperature correction (color tmp), which is an example of color correction, edge enhancement (sharpness), and contrast correction (contrast) can be selected via a menu for the predetermined image processing (image proc menu) in user interface display UI1 ((a), (b)). For example, when the color temperature correction (color tmp) is selected ((c)), three-choice selection of 5000K, 6000K, or 7000K is enabled. Then, if 5000K is selected ((d)), for example, an image obtained by performing color temperature correction of 5000K on an overhead-view image (an image illustrating a general view of the pathological image) is displayed in a pop-up manner as an example in region R12 in FIG. 9(a). The observer views this pop-up indication and if satisfied, performs an operation of confirming the selection so that color temperature correction of 5000K is performed on the entire display screen.

In this manner, the observer can easily select the details of the predetermined image processing on the pathological image by using the UI as described above. Note that, in the above-described example, the required processing time is reduced by performing the color temperature correction on the overhead-view image having a smaller area, instead of the entire display screen illustrated in FIG. 9(a), for display as an example.

FIG. 10 is a diagram schematically illustrating an example of display for identifying an unprocessed portion in a pathological image according to the first embodiment. As described above, the output processing procedure may also indicate that the unprocessed portion is displayed by means of a diagrammatic indication on the display unit 45. In the pathological image in FIG. 10, the inside of region R14 is a processed portion, and the outside of region R14 is an unprocessed portion. Also, region R13 is an overhead-view image. Also, icon A1 is an icon indicating that the unprocessed portion is displayed by displaying a sandglass. Also, status bar SB1 is a status bar indicating that the unprocessed portion is displayed by displaying an approximate remaining time.

In this manner, by indicating that the unprocessed portion is displayed by means of a diagrammatic indication with icon A1 or status bar SB1 in addition to the boundary line between the inside and outside of region R14, the observer can more reliably recognize that the unprocessed portion is present in the displayed pathological image.

Note that, although both of icon A1 and status bar SB1 are displayed in FIG. 10 for convenience of creating the drawings, there is no limitation in this regard, and only one of them may be displayed. Another illustrative indication may also be used.

In this manner, according to the diagnosis support system 1 in the first embodiment, by outputting the identification information for identifying the processed portion and the unprocessed portion of the image processing on the pathological image, the accuracy of the pathological diagnosis using the pathological image can be improved.

Specifically, by displaying a boundary line at the boundary between the processed portion and the unprocessed portion in the displayed pathological image, the observer can reliably distinguish the processed portion and the unprocessed portion in the pathological image, and a wrong diagnosis can be avoided.

Also, by highlighting the new processed portion by means of at least one of the color, thickness, or flashing of the boundary line surrounding it the observer reliably recognizes the new processed portion in the pathological image, and a wrong diagnosis can be better avoided.

Also, performing the image processing from a central portion of the displayed pathological image is convenient for the observer because it can be considered that the observer often views the pathological image from the central portion.

Also, when a cursor is displayed together with the pathological image, performing the image processing from a portion corresponding to the cursor position is convenient for the observer because it can be considered that the observer is likely to look at the cursor portion.

Also, by displaying the boundary line by lines of multiple line types, the observer can more reliably distinguish the processed portion and the unprocessed portion in the pathological image.

Also, by displaying the boundary line at the boundary between the processed portion and the unprocessed portion in the overhead-view image in enlarging a portion of the pathological image on the display unit 45, the observer can easily recognize which of the processed portion and the unprocessed portion is enlarged by viewing the overhead-view image.

Also, by highlighting (displaying with a bold frame or the like) the enlarged portion when it is the unprocessed portion in that process, the observer can further easily recognize that the enlarged portion is the unprocessed portion.

Also, by indicating at least one of the processed portion and the unprocessed portion of the pathological image by means of a text indication, the observer can more reliably identify the processed portion and the unprocessed portion.

Also, by indicating that the unprocessed portion is displayed by means of a diagrammatic indication (for example, icon A1 or status bar SB1 in FIG. 10), the observer can more reliably recognize that the unprocessed portion is present in the pathological image.

Also, by displaying the pathological image after performing the predetermined image-quality degradation processing (luminance reduction or gray-scaling) on the unprocessed portion, it is possible to further reduce the possibility of occurrence of a wrong diagnosis due to difficulty in viewing the unprocessed portion.

Note that the starting position of the predetermined image processing on the pathological image is not limited to the center of the screen or the cursor portion as described above and may be a lesion if the lesion is identified by estimation by machine learning or the like, for example.

Also, the predetermined image processing is not limited to color correction, edge enhancement, or contrast correction and may be other image processing such as tone curve correction.

### <Second Embodiment>

### [4. Configuration of System according to Second Embodiment]

Next, a diagnosis support system 1 according to a second embodiment will be described. Descriptions of items similar to those in the first embodiment will be omitted as appropriate. The second embodiment is different from the first embodiment in that what is called tile images are used. That is, in the second embodiment, a pathological image is composed of a plurality of tile images. Also, when the layer of tile images displayed on the display unit 45 is changed, the image processing procedure performs predetermined image processing on the tile images in the new layer. Details will be described below.

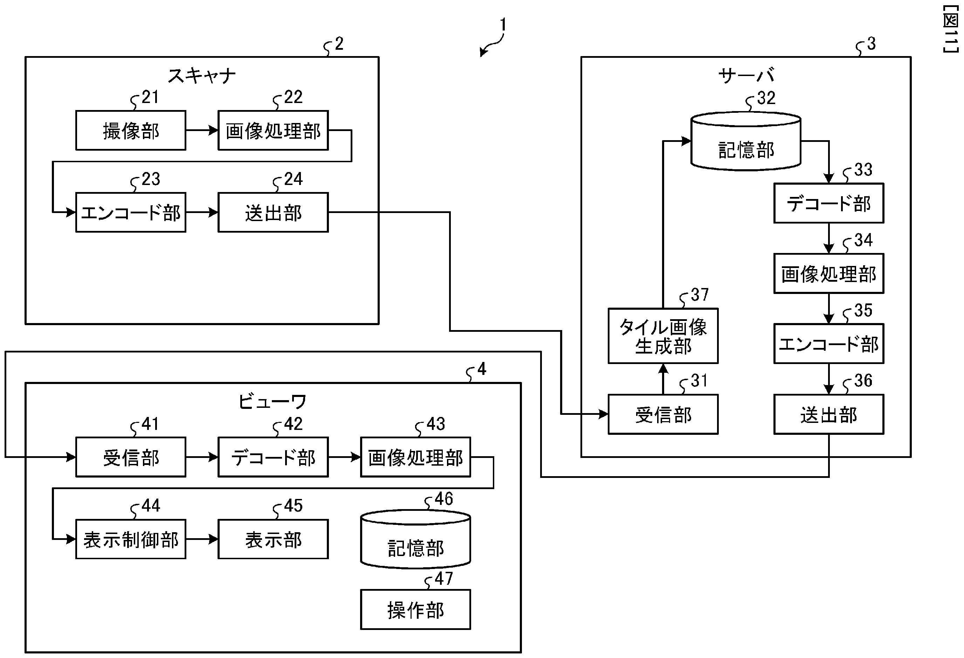

FIG. 11 is an entire configuration diagram of the diagnosis support system 1 according to the second embodiment. As compared to the case of the first embodiment, a tile image generating unit 37 is added to the server 3. The details of the tile image generating unit 37 will be described later.

### [5. Description of Tile Images in Second Embodiment]

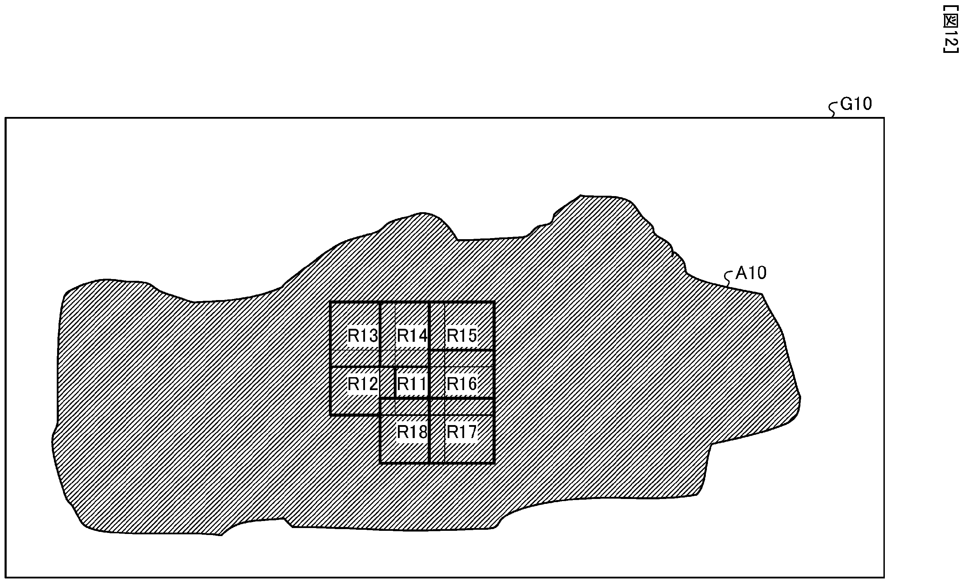

FIG. 12 is a diagram for illustrating an image-capturing process according to the second embodiment. As described above, the scanner 2 captures an image of an observation object A10 contained in a glass slide G10, and acquires a pathological image, which is a digital image.

In the second embodiment, for example, the scanner 2 generates an entire image and then identifies the region in which the observation object A10 is present in the entire image, and images of divided regions, obtained by dividing the region in which the observation object A10 is present by a predetermined size, are sequentially captured by a high-resolution image capturing unit. For example, as illustrated in FIG. 12, the scanner 2 first captures an image of region R11 and generates a high-resolution image I11, which is an image illustrating a partial region of the observation object A10. Subsequently, the scanner 2 moves the stage to capture an image of region R12 by the high-resolution image capturing unit and generates a high-resolution image 112 corresponding to region R12. The scanner 2 generates high-resolution images 113, 114,... corresponding to regions R13, R14,... in a similar manner. Although only regions up to R18 are illustrated in FIG. 12, the scanner 2 sequentially moves the stage to capture images of all divided regions corresponding to the observation object A10 by the high-resolution image capturing unit and generates high-resolution images corresponding to the respective divided regions.

Incidentally, the glass slide G10 may move on the stage when the stage is moved. If the glass slide G10 moves, a region of the observation object A10 for which image capture is not performed may occur. The scanner 2 performs image capture by the high-resolution image capturing unit such that adjacent divided regions partially overlap as illustrated in FIG. 12, so that the occurrence of a region for which image capture is not performed can be prevented even when the glass slide G10 moves a little.

Note that, although an example in which the image-capturing region is changed by moving the stage is shown above, the scanner 2 may change the image-capturing region by moving an optical system (such as the high-resolution image capturing unit). Also, in FIG. 12, there is illustrated an example in which the scanner 2 captures the images of the observation object A10 from its central portion. However, the scanner 2 may also capture the images of the observation object A10 in an order different from the order of image capture as illustrated in FIG. 12. For example, the scanner 2 may capture the images of the observation object A10 from its peripheral portions.

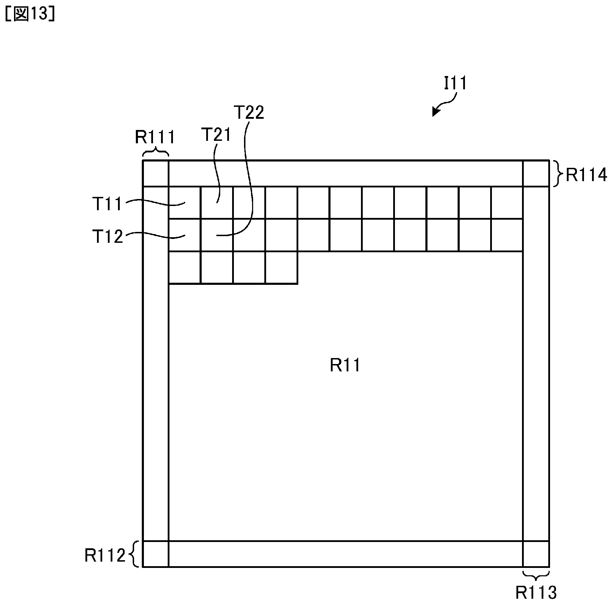

Subsequently, each high-resolution image generated by the scanner 2 is sent to the server 3 and divided by a predetermined size by the tile image generating unit 37 in the server 3. In this manner, partial images (tile images) are generated from the high-resolution image. This will be described by using FIG. 13. FIG. 13 is a diagram for illustrating a generation process of partial images (tile images) in the second embodiment.

FIG. 13 illustrates a high-resolution image I11 corresponding to region R11 illustrated in FIG. 12. Note that the following description will be made by assuming that partial images are generated from the high-resolution image by the server 3. However, partial images may also be generated by a device other than the server 3 (such as an information processing device provided in the scanner 2, for example).

In the example illustrated in FIG. 13, the server 3 divides the single high-resolution image I11 to generate 100 tile images T11, T12,.... For example, if the resolution of the high-resolution image I11 is 2560x2560 [pixels], the server 3 generates 100 tile images T11, T12,... with a resolution of 256x256 [pixels] from the high-resolution image I11. Similarly, the server 3 divides the other high-resolution images by the same size to generate tile images.

Note that, in the example of FIG. 13, regions R111, R112, R113, and R114 are regions overlapping other adjacent high-resolution images (not illustrated in FIG. 13). The server 3 performs alignment of the overlapping regions by a technique such as template matching to perform stitching processing on the high-resolution images adjacent to each other. In this case, the server 3 may generate tile images by dividing the high-resolution images after the stitching processing. Alternatively, the server 3 may generate tile images of regions other than region R111, R112, R113, and R114 before the stitching processing and generate tile images of region R111, R112, R113, and R114 after the stitching processing.

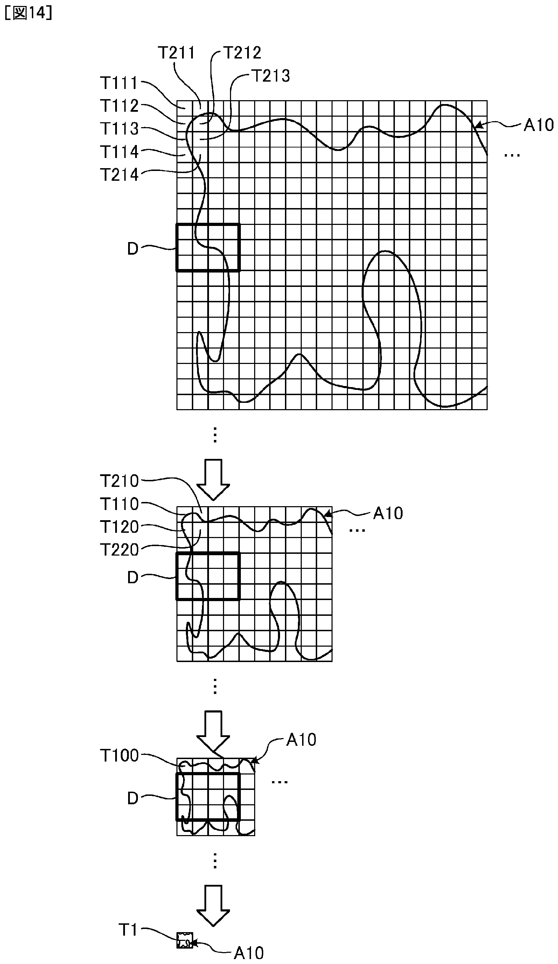

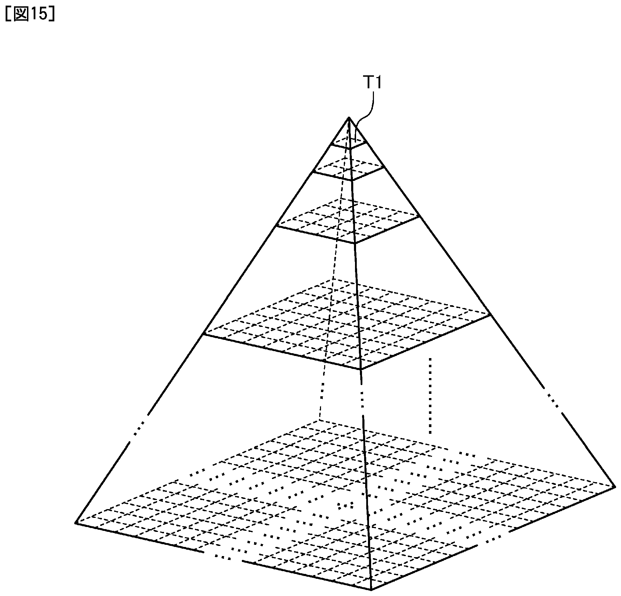

In this manner, the server 3 generates tile images as minimum units of the captured image of the observation object A10. The server 3 (or the viewer 4) then sequentially composites the tile images of minimum units to generate tile images with different layers. Specifically, the server 3 composites a predetermined number of adjacent tile images to generate one tile image. This will be described by using FIGS. 14 and 15. FIGS. 14 and 15 are diagrams for illustrating a pathological image according to the second embodiment.

In the upper part of FIG. 14, there is illustrated a group of tile images of minimum units generated from each high-resolution image by the server 3. In the example of the upper part of FIG. 14, the server 3 composites four tile images T111, T112, T211, and T212 adjacent to each other out of the tile images to generate one tile image T110. For example, if each of tile images T111, T112, T211, and T212 has a resolution of 256×256, the server 3 generates tile image T110 with a resolution of 256×256. Similarly, the server 3 composites four tile images T113, T114, T213, and T214 adjacent to each other to generate tile image T120. In this manner, the server 3 generates tile images compositing a predetermined number of tile images of minimum units for each.

The server 3 also generates tile images by further compositing tile images adjacent to each other out of the tile images obtained by compositing the tile images of minimum units. In the example of FIG. 14, the server 3 composites four tile images T110, T120, T210, and T220 adjacent to each other to generate one tile image T100. For example, if tile images T110, T120, T210, and T220 have a resolution of 256×256, the server 3 generates tile image T100 with a resolution of 256×256. For example, the server 3 generates tile images with a resolution of 256x256 by performing four-pixel averaging, a weighting filter (processing for increasing reflection of closer pixels relative to farther pixels), 1/2 thinning-out processing, or the like from images with a resolution of 512×512 obtained by compositing four tile images adjacent to each other.

The server 3 repeats such composition processing to finally generate one tile image having the same resolution as the resolution of the tile images of minimum units. For example, as in the above-described example, if the tile images of minimum units have a resolution of 256×256, the server 3 repeats the above-described composition processing to finally generate one tile image T1 with a resolution of 256×256.

FIG. 15 schematically illustrates the tile images illustrated in FIG. 14. In the example illustrated in FIG. 15, the group of tile images in the lowest layer is the tile images of minimum units generated by the server 3. The group of tile images in the second layer from the bottom is tile images obtained by compositing the group of tile images in the lowest layer. Tile image T1 in the uppermost layer represents one tile image finally generated. In this manner, the server 3 generates, as the pathological image, groups of tile images having layers such as a pyramid structure as illustrated in FIG. 15.

Note that region D illustrated in FIG. 14 represents an example of a region displayed on a display screen such as the display unit 45. For example, it is assumed that the resolution that can be displayed by the display device is three tile images vertically by four tile images horizontally. In this case, as in region D illustrated in FIG. 14, the degree of detailedness of the observation object A10 displayed on the display device depends on the layer to which the tile images being displayed belong. For example, when the tile images in the lowest layer is used, a small region of the observation object A10 is displayed in detail. As the tile images used are in a higher layer, a larger region of the observation object A10 is displayed more coarsely.

The server 3 stores the tile images in each layer as illustrated in FIG. 15 in the storage unit 32. For example, the server 3 stores each tile image together with tile identification information (an example of partial image information) capable of uniquely identifying each tile image. In this case, when accepting a request for acquiring a tile image including tile identification information from the viewer 4, the server 3 sends the tile image corresponding to the tile identification information to the viewer 4. Also, for example, the server 3 may store each tile image together with layer identification information that identifies each layer and tile identification information capable of unique identification within the same layer. In this case, when accepting a request for acquiring a tile image including layer identification information and tile identification information from the viewer 4, the server 3 sends, to the viewer 4, the tile image corresponding to the tile identification information out of the tile images belonging to the layer corresponding to the layer identification information.

Note that the server 3 may also store the tile images in each layer as illustrated in FIG. 15 in the viewer 4, a cloud server (not illustrated), or the like. Also, the generation process of the tile images illustrated in FIGS. 14 and 15 may be performed in the cloud server or the like.

Also, the server 3 may not store the tile images in all layers. For example, the server 3 may store only the tile images in the lowest layer, may store only the tile images in the lowest layer and the tile image in the uppermost layer, or may store the tile images in predetermined layers (such as odd-numbered layers or even-numbered layers, for example). At this time, when a tile image in a layer not stored is requested from another device, the server 3 dynamically composites stored tile images to generate the tile image requested from the other device. In this manner, for the server 3, it is possible to prevent a squeeze on the storage capacity by reducing the tile images to be stored.

Although image-capturing conditions are not mentioned in the above-described example, the server 3 may store the tile images in each layer as illustrated in FIG. 15 for each image-capturing condition. An example image-capturing condition is the focal length to an object (such as the observation object A10). For example, the scanner 2 may capture images while changing the focal length to the same object. In this case, the server 3 may store the tile images in each layer as illustrated in FIG. 15 for each focal length. Note that the reason for changing the focal length is that the observation object A10 may be semitransparent and thus there are a focal length suitable for capturing an image of the surface of the observation object A10 and a focal length suitable for capturing an image of the inside of the observation object A10. In other words, the scanner 2 can generate a pathological image captured from the surface of the observation object A10 and a pathological image captured from the inside of the observation object A10 by changing the focal length.

Another example image-capturing condition is a condition of staining the observation object A10. To specifically describe, in pathological diagnosis, a particular portion of the observation object A10 (such as a cell, for example) may be stained with a light-emitting substance. The light-emitting substance refers to a substance that emits light in response to irradiation of light of a particular wavelength, for example. The same observation object A10 may be stained with different light-emitting substances. In this case, the server 3 may store the tile images in each layer as illustrated in FIG. 15 for each light-emitting substance used for the staining.

Also, the number of tile images and the resolution described above are an example and can be changed as appropriate to the system. For example, the number of tile images composited by the server 3 is not limited to four. For example, the server 3 may repeat a process of compositing 3 × 3 = 9 tile images. Also, although an example in which the resolution of the tile images is 256x256 is shown above, the resolution of the tile image may be other than 256×256.

The viewer 4 uses software adopting a system capable of handling the groups of tile images with the layer structure described above, extracts a desired tile image from the groups of tile images with the layer structure according to an input operation of the user, and outputs it on the display unit 45. Specifically, the display unit 45 displays an image of a certain portion selected by the user in an image with a certain resolution selected by the user. Such processing allows the user to experience a feeling of observing the observation object while changing the observation magnification. That is, the viewer 4 functions as a virtual microscope. The virtual observation magnification here actually corresponds to the resolution.

### [6. Example of Displaying Pathological Image according to Second Embodiment]

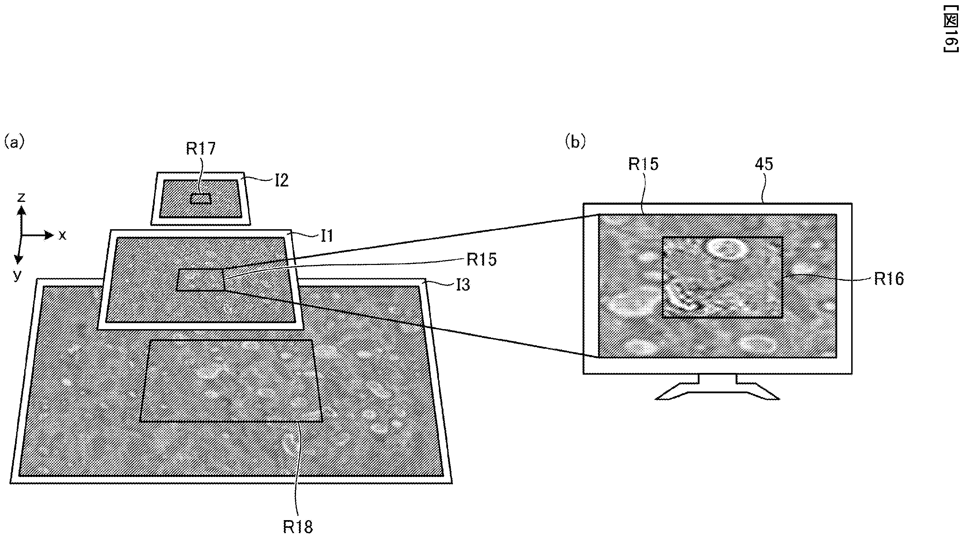

FIG. 16 is a diagram schematically illustrating an example of displaying a pathological image according to the second embodiment. Pathological image I1 illustrated in FIG. 16(a) is composed of a group of tile images in a middle layer portion of the pyramid structure. Region R15 of pathological image I1 is displayed on the display unit 45, as illustrated in FIG. 16(b). In the pathological image in FIG. 16(b), the inside of region R16 is a processed portion, and the outside of region R16 is an unprocessed portion. A boundary line is displayed at their boundary.

Also, pathological image I2 is composed of a group of tile images in a higher layer than pathological image I1. Region R17 of pathological image I2 corresponds to region R15 of pathological image I1. Also, pathological image I3 is composed of a group of tile images in a lower layer than pathological image I1. Region R18 of pathological image I3 corresponds to region R15 of pathological image I1.

In this case, for example, while region R15 of pathological image I1 is displayed, the predetermined image processing may be performed on background on region R17 of pathological image I2 and region R18 of pathological image I3 corresponding to it to reduce the time to make the user wait during a zooming operation (Z-direction movement). The predetermined image processing may also be performed in advance by predicting user operations for X-direction and Y-direction.

In this manner, according to the diagnosis support system 1 in the second embodiment, by outputting the identification information (for example, displaying the boundary line) for identifying the processed portion and the unprocessed portion of the image processing even for a pathological image composed of a plurality of tile images, the accuracy of the pathological diagnosis using the pathological image can be improved.

Specifically, for example, when the layer of tile images displayed is changed, by performing the predetermined image processing on the tile images in the new layer and performing the display of the boundary line between the processed portion and the unprocessed portion or the like, the observer can reliably distinguish the processed portion and the unprocessed portion in the pathological image.

### <Third Embodiment>

### [7. Configuration of System according to Third Embodiment]

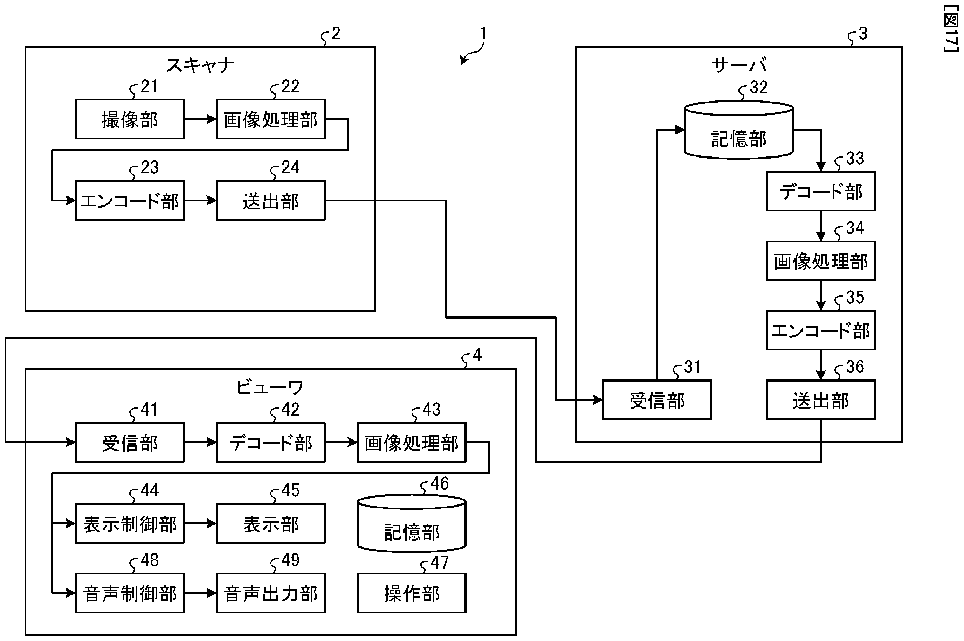

Next, a diagnosis support system 1 according to a third embodiment will be described. Descriptions of items similar to those in the first embodiment will be omitted as appropriate. FIG. 17 is an entire configuration diagram of the diagnosis support system 1 according to the third embodiment. The third embodiment is different from the first embodiment in that a sound control unit 48 and a sound output unit 49 are added to the viewer 4.

The sound control unit 48 performs an output processing procedure of outputting a sound as identification information for identifying a processed portion and an unprocessed portion of the image processing on the pathological image. The sound output unit 49 is a means for outputting a sound and is, for example, a speaker. For example, when the unprocessed portion is displayed on the display unit 45, the sound control unit 48 makes a sound notification to that effect.

In this manner, according to the diagnosis support system 1 in the third embodiment, not only display but also a sound can be used for identifying the processed portion and the unprocessed portion of the image processing on the pathological image. Therefore, the observer can more reliably distinguish the processed portion and the unprocessed portion in the pathological image.

### <Other Embodiments>

The processes according to the above-described embodiments may be performed in various different forms other than the above-described embodiments.

### [Display Device]

In the above-described embodiments, it is assumed that the display unit 45 is a display device of a desktop type. However, there is no limitation in this regard, and the display unit 45 may also be a wearable device (such as a head-mounted display) worn by the pathologist or the like.

### [Image-Capturing Device]

Also, although description is made by using a scanner as an example of the device for capturing images of the observation object in the above-described embodiments, there is no limitation in this regard. For example, the device for capturing images of the observation object may be a medical image acquisition device such as an endoscope, computed tomography (CT), or magnetic resonance image (MRI) for capturing images of the inside of the body of a patient. In this case, medical images such as two-dimensional static images or moving images generated by the endoscope or three-dimensional images generated by the CT or MRI are saved in the server 3.

### [Server]

Other pathological images captured by another medical image acquisition device such as an endoscope, CT, or MRI may also be stored in the server 3 in association with the pathological images generated by the scanner 2.

### [Hardware Configuration]

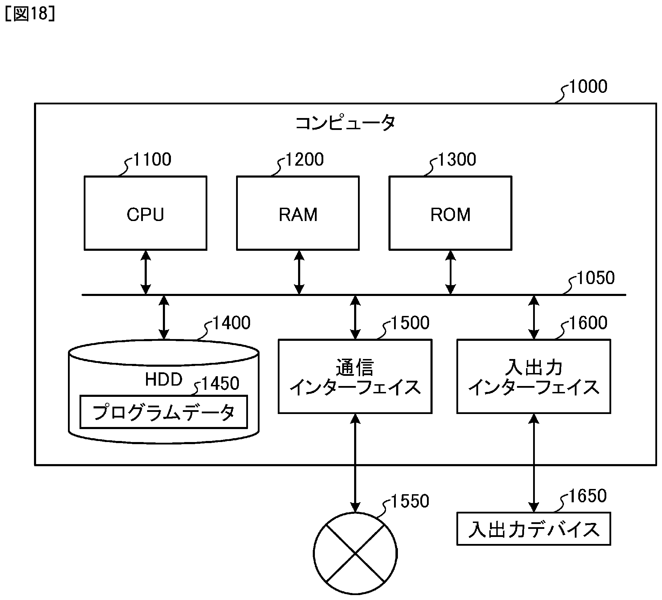

Information equipment such as the server 3 or the viewer 4 according to the above-described embodiments is realized by a computer 1000 having a configuration as illustrated in FIG. 18, for example. The following will describe an example of the viewer 4 according to the first embodiment. FIG. 18 is a hardware configuration diagram illustrating an example of a computer for realizing functions of the viewer 4.

The computer 1000 includes a CPU 1100, a RAM 1200, a read only memory (ROM) 1300, a hard disk drive (HDD) 1400, a communication interface 1500, and an input/output interface 1600. The components of the computer 1000 are connected by a bus 1050.

The CPU 1100 operates based on programs stored in the ROM 1300 or the HDD 1400 and controls the components. For example, the CPU 1100 develops the programs stored in the ROM 1300 or the HDD 1400 onto the RAM 1200 and performs processing corresponding to the various programs.

The ROM 1300 stores a boot program such as a basic input output system (BIOS) executed by the CPU 1100 at the time of starting the computer 1000, programs depending on the hardware of the computer 1000, and the like.

The HDD 1400 is a computer-readable recording medium that non-transitorily records programs executed by the CPU 1100, data used by the programs, and the like. Specifically, the HDD 1400 is a recording medium that records a response-generating program according to the present disclosure, which is an example of program data 1450.

The communication interface 1500 is an interface for the computer 1000 connecting to an external network 1550 (for example, the Internet). For example, the CPU 1100 receives data from other equipment and sends data generated by the CPU 1100 to other equipment via the communication interface 1500.

The input/output interface 1600 is an interface for connecting input/output devices 1650 and the computer 1000. For example, the CPU 1100 receives data from input devices such as a keyboard or a mouse via the input/output interface 1600. The CPU 1100 also sends data to output devices such as a display, a speaker, or a printer via the input/output interface 1600. The input/output interface 1600 may also function as a media interface that reads programs recorded in a predetermined computer-readable recording medium (media) and the like. Examples of the media include optical recording media such as a digital versatile disc (DVD) and a phase change rewritable disk (PD), magneto-optical recording media such as a magneto-optical disk (MO), tape media, magnetic recording media, semiconductor memories, and the like.

For example, if the computer 1000 functions as the viewer 4 according to the first embodiment, the CPU 1100 of the computer 1000 executes a diagnosis support program loaded on the RAM 1200 to realize the functions of the receiving unit 41, the decoding unit 42, the image processing unit 43, the display control unit 44, and the like.

### [Others]

All or some of the processes described as being performed automatically in the above-described embodiments can also be performed manually, or all or some of the processes described as being performed manually can also be performed automatically in a known method. Besides, information including processing procedures, specific names, various data and parameters illustrated in the above text and the drawings can be modified as desired unless otherwise specified. For example, various pieces of information illustrated in the drawings are not limited to the illustrated information.

Also, the components of the devices illustrated are functional concepts and are not necessarily required to be physically configured as illustrated. That is, the specific forms of distribution and integration of the devices are not limited to those illustrated, and all or some of them can be functionally or physically distributed or integrated in any units according to various loads, usage conditions, and the like.

Also, the embodiments and variations described above can be combined as appropriate without inconsistency of processes.

Note that the effects described herein are merely an example and not limiting, and other effects may be provided.

Note that the present technique can take the following configurations.
(1) A diagnosis support program for causing a computer to perform:
   an image processing procedure of performing predetermined image processing on a pathological image captured by an image-capturing device; and
   an output processing procedure of outputting identification information for identifying a processed portion and an unprocessed portion of the image processing on the pathological image.
(2) The diagnosis support program according to (1), wherein
   the output processing procedure comprises:
   displaying a boundary line as the identification information at a boundary between the processed portion and the unprocessed portion displayed on a display unit.
(3) The diagnosis support program according to (2), wherein
   the output processing procedure comprises:
   highlighting a new processed portion of the processed portion displayed on the display unit by means of at least one of a color, a thickness, or flashing of a boundary line surrounding it.
(4) The diagnosis support program according to (2) or (3), wherein
   the image processing procedure comprises:
   performing the image processing from a central portion of a region displayed on the display unit in the pathological image.
(5) The diagnosis support program according to (2) or (3), wherein
   the image processing procedure comprises:
   performing the image processing from a portion corresponding to a cursor position displayed on the display unit in the pathological image.
(6) The diagnosis support program according to (2) or (3), wherein
   the output processing procedure comprises:
   displaying the boundary line by a line of a first line type positioned closer to the processed portion and a line of a second line type positioned closer to the unprocessed portion.
(7) The diagnosis support program according to (2) or (3), wherein
   the output processing procedure comprises:
   displaying the entire pathological image in a portion of the display unit and displaying a boundary line at a boundary between the processed portion and the unprocessed portion in the entire displayed pathological image in enlarging a portion of the pathological image on the display unit.
(8) The diagnosis support program according to (7), wherein
   the output processing procedure comprises:
   highlighting a portion of the pathological image enlarged on the display unit when the portion of the pathological image is the unprocessed portion.
(9) The diagnosis support program according to (2) or (3), wherein
   the output processing procedure comprises:
   indicating at least one of the processed portion and the unprocessed portion by means of a text indication on the display unit.
(10) The diagnosis support program according to (2) or (3), wherein
   the output processing procedure comprises:
   indicating that the unprocessed portion is displayed by means of a diagrammatic indication on the display unit.
(11) The diagnosis support program according to (2) or (3), wherein
   the output processing procedure comprises:
   performing display on the display unit after performing predetermined image-quality degradation processing on the unprocessed portion.
(12) The diagnosis support program according to (2) or (3), wherein
   the pathological image is composed of a plurality of tile images, and
   the image processing procedure comprises:
      performing the predetermined image processing on a tile image in a new layer when a layer of the tile images displayed on the display unit is changed.
(13) The diagnosis support program according to (2) or (3), wherein
   the output processing procedure comprises:
   when the unprocessed portion is displayed on the display unit, making a sound notification to that effect.
(14) A diagnosis support system comprising:
   an image-capturing device; and
   an information processing device that performs predetermined image processing on a pathological image captured by the image-capturing device and outputs identification information for identifying a processed portion and an unprocessed portion of the image processing on the pathological image.
(15) A diagnosis support method in which a computer performs:
   an image processing procedure of performing predetermined image processing on a pathological image captured by an image-capturing device; and
   an output processing procedure of outputting identification information for identifying a processed portion and an unprocessed portion of the image processing on the pathological image.
(16) A diagnosis support system comprising an image-capturing device and software used for processing a pathological image captured by the image-capturing device, wherein
   the software is software for causing an information processing device to perform image processing for performing predetermined image processing on the pathological image and output processing for outputting identification information for identifying a processed portion and an unprocessed portion of the image processing on the pathological image.

### Reference Signs List

- 1: DIAGNOSIS SUPPORT SYSTEM

- 2: SCANNER
- 3: SERVER
- 4: VIEWER
- 21: IMAGE CAPTURING UNIT
- 22: IMAGE PROCESSING UNIT
- 23: ENCODING UNIT
- 24: SENDING UNIT
- 31: RECEIVING UNIT
- 32: STORAGE UNIT
- 33: DECODING UNIT
- 34: IMAGE PROCESSING UNIT
- 35: ENCODING UNIT
- 36: SENDING UNIT
- 37: TILE IMAGE GENERATING UNIT
- 41: RECEIVING UNIT
- 42: DECODING UNIT
- 43: IMAGE PROCESSING UNIT
- 44: DISPLAY CONTROL UNIT
- 45: DISPLAY UNIT
- 46: STORAGE UNIT
- 47: OPERATION UNIT
- 48: SOUND CONTROL UNIT
- 49: SOUND OUTPUT UNIT

## Claims

1. A diagnosis support program for causing a computer to perform:
an image processing procedure of performing predetermined image processing on a pathological image captured by an image-capturing device; and
an output processing procedure of outputting identification information for identifying a processed portion and an unprocessed portion of the image processing on the pathological image.

2. The diagnosis support program according to claim 1, wherein
the output processing procedure comprises:
displaying a boundary line as the identification information at a boundary between the processed portion and the unprocessed portion displayed on a display unit.

3. The diagnosis support program according to claim 2, wherein
the output processing procedure comprises:
highlighting a new processed portion of the processed portion displayed on the display unit by means of at least one of a color, a thickness, or flashing of a boundary line surrounding it.

4. The diagnosis support program according to claim 2, wherein
the image processing procedure comprises:
performing the image processing from a central portion of a region displayed on the display unit in the pathological image.

5. The diagnosis support program according to claim 2, wherein
the image processing procedure comprises:
performing the image processing from a portion corresponding to a cursor position displayed on the display unit in the pathological image.

6. The diagnosis support program according to claim 2, wherein
the output processing procedure comprises:
displaying the boundary line by a line of a first line type positioned closer to the processed portion and a line of a second line type positioned closer to the unprocessed portion.

7. The diagnosis support program according to claim 2, wherein
the output processing procedure comprises:
displaying the entire pathological image in a portion of the display unit and displaying a boundary line at a boundary between the processed portion and the unprocessed portion in the entire displayed pathological image in enlarging a portion of the pathological image on the display unit.

8. The diagnosis support program according to claim 7, wherein
the output processing procedure comprises:
highlighting a portion of the pathological image enlarged on the display unit when the portion of the pathological image is the unprocessed portion.

9. The diagnosis support program according to claim 2, wherein
the output processing procedure comprises:
indicating at least one of the processed portion and the unprocessed portion by means of a text indication on the display unit.

10. The diagnosis support program according to claim 2, wherein
the output processing procedure comprises:
indicating that the unprocessed portion is displayed by means of a diagrammatic indication on the display unit.

11. The diagnosis support program according to claim 2, wherein
the output processing procedure comprises:
performing display on the display unit after performing predetermined image-quality degradation processing on the unprocessed portion.

12. The diagnosis support program according to claim 2, wherein
the pathological image is composed of a plurality of tile images, and
the image processing procedure comprises:
performing the predetermined image processing on a tile image in a new layer when a layer of the tile images displayed on the display unit is changed.

13. The diagnosis support program according to claim 2, wherein
the output processing procedure comprises:
when the unprocessed portion is displayed on the display unit, making a sound notification to that effect.

14. A diagnosis support system comprising:
an image-capturing device; and
an information processing device that performs predetermined image processing on a pathological image captured by the image-capturing device and outputs identification information for identifying a processed portion and an unprocessed portion of the image processing on the pathological image.

15. A diagnosis support method in which a computer performs:
an image processing procedure of performing predetermined image processing on a pathological image captured by an image-capturing device; and
an output processing procedure of outputting identification information for identifying a processed portion and an unprocessed portion of the image processing on the pathological image.

16. A diagnosis support system comprising an image-capturing device and software used for processing a pathological image captured by the image-capturing device, wherein
the software is software for causing an information processing device to perform image processing for performing predetermined image processing on the pathological image and output processing for outputting identification information for identifying a processed portion and an unprocessed portion of the image processing on the pathological image.
